# EUROPEAN PATENT APPLICATION

(11) **EP 2 030 619 A1**
(43) Date of publication of application: **04.03.2009**
(21) Application number: 07110478.0
(22) Date of filing: 18.06.2007
(51) Int. Cl.: A61K 31/435, A61P 25/28

(54) **Use of dopamine stabilizers**

(71) Applicant: A.Carlsson Research AB, 413 19 Göteborg (SE)
(72) Inventor: Carlsson, Arvid, 413 19, Göteborg (SE); Tamminga, Carol A., Dallas, TX 75209 (US); Lahti, R. A., deceased (US)
(74) Representative: Stenbäck, Maria Elisabeth

(57) **Abstract**

The present invention relates to the use of a dopamine stabilizing substance in an oral, subcutaneous or intramuscular daily dose of 1-20 mg or in an intravenous daily dose of 0.1-2 mg in treatment of a neurological or psychiatric disorder characterized by a hypofunction of the dopamine system. The invention also relates to the use of a dopamine stabilizing substance in an oral, subcutaneous or intramuscular daily dose of 25-50 mg in treatment of a disorder caused by instability of neural circuits

## Description

### Background

Dopamine stabilizers is a class of compounds which have the ability to reverse both hypo as well as hyperdopaminergia in vivo. This class may be exemplified by the phenylpiperidines (-)-OSU6162 and ACR16.
The compound (-)-OSU6162 (S-(-)-3-[3-methylsulfonylphenyl]-1-propylpiperidine) belongs to a group of phenylpiperidines developed in the Department of Pharmacology, University of Gothenburg, Sweden, in collaboration with The Upjohn Company, Kalamazoo, Michigan, USA (Sonesson et al. 1994). When characterized in animal in-vivo models this group of agents showed a profile similar to the partial dopamine D₂/D₃ receptor agonist (-)-3-PPP (S-(-)-3-[3-hydroxyphenyl]-1-propylpiperidine). This was especially true in regard to behaviour, in that not only inhibitory but also weakly stimulatory actions could be demonstrated, depending on the baseline activity level. Clearly the tendency to immobility ("catalepsy") even after high doses is weak (Natesan et al 2006), and extrapyramidal side effects did not occur in clinical studies demonstrating an antipsychotic action (Gefvert et al 2000, L. Lindström, unpublished data, Carlsson and Carlsson 2006). In these respects these agents are similar to (-)-3-PPP. Moreover, they show albeit moderate affinity for dopamine D₂ receptors, when studied in vitro, and their binding to these receptors is clearly documented in animal ex-vivo experiments. However, (-)-OSU6162 and its congeners did not show any signs of intrinsic activity on dopamine receptors in in-vivo or ex-vivo models (Sonesson et al 1994). These compounds, like (-)-3-PPP, are phenylpiperidines, but they differ from this agent in having electron-drawing substituents in 3-position on the phenyl ring rather than the OH group of (-)-3-PPP. The OH group has been assumed to be important for the partial and nearly full dopamine D₂/D₃-receptor agonist properties of (-)-3-PPP and (+)-3PPP, respectively.
In the absence of partial agonism the somewhat similar behavioural profile of (-)-OSU6162 and its congeners to (-)-3-PPP calls for an explanation. So far, attempts to clarify this issue have been based on the assumption of heterogeneity of dopamine D2 receptors (see Carlsson et al 2004, Carlsson and Carlsson 2006).
The substance (-)-OSU6162, or S-(-)-3-[3-methylsulfonylphenyl]-1-propylpiperidine), has so far been used in clinical dosages of at least 25 mg per day, and often higher. One major disadvantage is that it has been found that such high dosages may be connected to QTc values higher than normal, cardiac arrhythmia, or heart rhythm abnormalities, such as torsades de pointes.

### Summery of the invention

The present invention is based on the finding of the stimulating and inhibitory effects of the dopamine "stabilizer" (-)-OSU6162 on dopamine D₂ receptor function in vitro. The present invention is defined in the appended claims.

### Brief description of the drawings

Figure 1. Incorporation of GTPgammaS³⁵ in CHO-hD₂ₗ membranes in the presence of various concentrations of dopamine and (-)-OSU 6162. Overview of the entire experiment. For statistics, see legends to Figures 2 and 3.
Figure 2. Incorporation of GTPgammaS³⁵ in CHO-hD₂ₗ membranes following exposure to various concentrations of (-)-OSU 6162 in the absence of dopamine.
   Data are means and SD, expressed as cpm (zero values N=6, other groups N=3). ANOVA shows significant differences among treatment groups (F_{(4,12)}=4.76, P=0.0156). Zero values differ from 100 (P=0.0361), 1,000 (P=0.0067), and 10,000 nM (P=0.0032) concentrations of (-)-OSU 6162 . Differences between other groups are not significant.

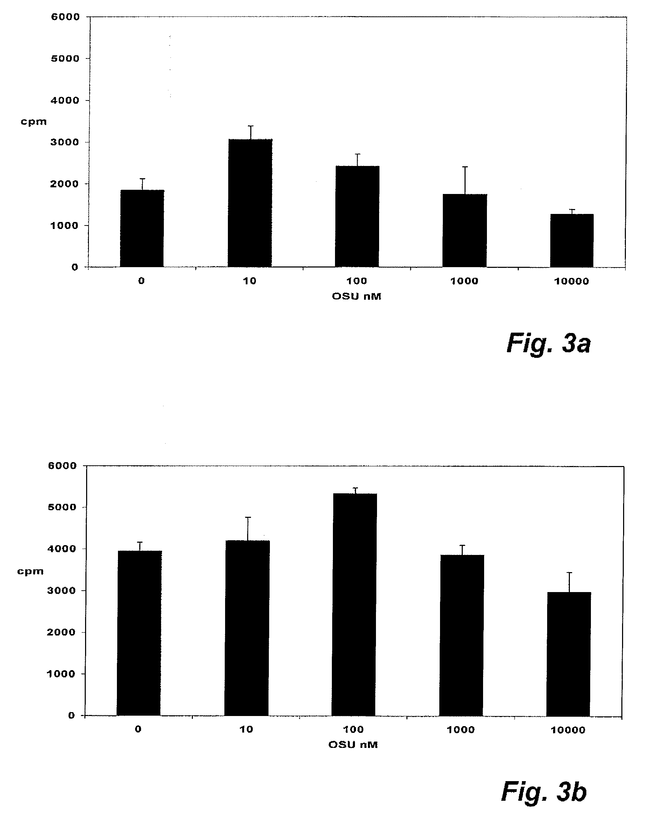

Figure 3. Incorporation of GTPgammaS³⁵ in CHO-hD₂ₗ membranes in the presence of two concentrations of dopamine (10 nM, Fig 3a, and 100 nM, Fig 3b) and various concentrations of (-)-OSU 6162.
   Data are means and SD of triplicates. They were obtained following subtraction from blank values, recorded in the absence of dopamine and (-)-OSU 6162. The blanks were 8,291±281 cpm (means±SD, n=6).
   For either dopamine concentration (10 nM, in Fig 3a; 100 nM, iin Fig 3b) ANOVA showed significant differences among the treatment groups, F(_{4,10})=4.36 and 16.05, respectively. In the 10 nM dopamine concentration series 10 nM (-)-OSU 6162 differed from zero (P=0.0246) and 10 nM (-)-OSU6162 differed from 1000 nM (p=0.0176 and 10000 nM (p=0.0033) (Fig 3a). In the 100 nM dopamine concentration series 100 nM (-)-OSU 6162 yielded values significantly greater than all the 4 other treatment groups (P=<0.003 in each case) (Fig. 3b), confirming a biphasic concentration-response curve.

### Detailed description of the invention

As already explained above, attempts to clarify the somewhat similar behavioural profile of (-)-OSU6162 and its congeners to (-)-3-PPP in the absence of partial agonism have so far been based on the assumption of heterogeneity of dopamine D₂ receptors. The inventors of the present invention have now found an additional, but not necessarily alternative, explanation. This is further explained below, i.a. by the in-vitro experiments on cloned dopamine D₂ receptors.
The inventors have found that low concentrations of a dopamine stabilizing compound such as (-)-OSU6162 enhances the stimulating action of dopamine, suggesting a weak partial agonism. However, this enhancing effect is reversed by higher concentrations of the dopamine stabilizing compound such (-)-OSU6162 in a complex biphasic manner. The dopamine-enhancing action is proposed to be mediated by binding to an allosteric site with high affinity and the inhibitory component by a low-affinity binding to the orthosteric site of the dopamine receptor.
The present invention relates to the medicinal use of a dopamine stabilizing substance, also called dopaminergic stabilizer or dopamine stabilizer, in a low dose, such as 1-20 mg.
The dopamine stabilizing substance may be a compound of formula I 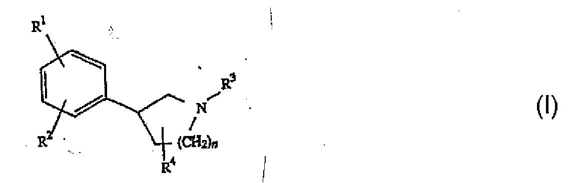 wherein:
- R¹ and R²: are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
- R³: is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
- R⁴ and R: are independently selected from hydrogen, CF₃.CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or-(CH₂)ₘ-R⁵ where m is 1-8;
- R⁵: is phenyl, phenyl substituted with CN, CF₃, CH₂CF₃, C₁ -C₈ alkyl, C₃ - C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷; and
- R⁶ and R⁷: are independently H, C₁-C₈ alkyl, C₃-C₈ cycloalkyl, C₄-C₉ cycloalkyl-methyl, C₂-C₈ alkenyl or C₂-C₈ alkynyl;
with the provisos that (i) when R¹ is CN, R² and R⁴ are H, and R³ is n-Pr then the compound is a pure enantiomer: and (ii) when R¹ or R² is OH, or CONH₂ then R⁴ is not H or methyl

Dopamine stabilizing substances of formula I and pharmaceutically acceptable salts thereof have been described in US 5,462,947, the teachings of which are hereby incorporated by reference. US 5,462,947 discloses the compounds belonging to this group and also gives the definitions for the different terms used - see in particular column 4. line 54 - column 6, line 25. US 5,462,947 also discloses how these compounds may be obtained - see in particular column 7, lines 26-28 and the Examples.

According to one embodiment, a compound of formula I in the form of a pure enantiomer or a pharmaceutically acceptable salt thereof is used.
According to one embodiment a compound of formula I wherein R¹ is CN, OSO₂CF₃, or SO₂CH₃ or a pharmaceutically acceptable salt thereof is used. It may then be preferable that R² is H and R³ is C₁₋₈ alkyl, and further that R³ is n-propyl, and moreover that R⁴ is H.
According to one embodiment a compound of formula I wherein R¹ is 3-OH, R² is H, R³ is n-propyl and R⁴ is C₂₋₈ alkyl or a pharmaceutically acceptable salt thereof is used.
According to one embodiment the compound of formula I is S-(-)-3-[3-methylsulfonylphenyl]-1-propylpiperidine).
The dopamine stabilizing substance may also be a compound of formula II 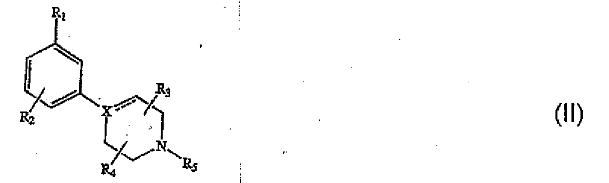 wherein
- R₁: is selected from the group consisting of OSO₂CF₃, OSO₂CH₃, SOR₃, SO₂R₃, COCH₃, and COCH₂CH₃, wherein R₃ is as defined below;
- R₂: is selected from the group consisting of C₂-C₄ branched or unbranched alkyls, terminal allyl, CH₂CH₂OCH₃, CH₂CH₂CH₂F, CH₂CF₃, 3,3,3-trifluoropropyl, and 4,4,4-trifluorobutyl,
- R₃: is selected from the group consisting of C1-C₃ alkyls, CF₃, and N(CH₃)₂;.
wherein the compound of formula II does not have a high binding affinity to sigma receptors

Dopamine stabilizing substances of formula II and pharmaceutically acceptable salts thereof, have been described in US 6,903,120, the teachings of which are hereby incorporated by reference.
According to one embodiment a compound of formula II wherein R₁ is selected from the group consisting of OSO₂CF₃, OSO₂CH₃, SO₂CH₃, SO₂CF₃, COCH₃, and SO₂N(CH₃)₂ or a pharmaceutically acceptable salt thereof is used. It may then be preferable that R₁ is selected from the group consisting of SO₂CF₃, SO₂CH₃, and COCH₃.
According to one embodiment a compound of formula II wherein R₂ is selected from the group consisting of n-propyl and ethyl or a pharmaceutically acceptable salt thereof is used.
According to one embodiment the compound of formula II is 4-(3-methanesulfonylphenyl)-1-propyl-piperidine.
The dopamine stabilizing substance may also be a pharmaceutically acceptable salts of a compound of formula I and II.
Furthermore, it is possible to use a combination of two or more of the dopamine stabilizing substances according to the invention, provided that the total dosage is still within the limits of the low dosage according to the invention.
The clinical implications of the invention are at least twofold.
First, the discovery that the dopamine stabilizers described in the invention, can bind with high affinity to a putative allosteric site on the dopamine D2 receptor, leading to a stimulation of dopamine function, opens up new possibilities for treatment of a variety of neurological and psychiatric disorders characterized by a hypofunction of the dopamine system. In such cases the dosage of the stabilizer should be kept low to match the approximately 50 nanomolar concentrations needed for an adequate response, as further shown below. Although it is not possible to translate in-vitro data precisely to in-vivo dosages, it is reasonable to assume that the doses used so far, which were intended to match the micromolar affinities of the stabilizers to the orthosteric site of the same receptor, are severalfold higher than those needed to stimulate dopamine function via binding to the allosteric site. The oral doses used so far have been in the order of 25 to 150 mg per day. The doses needed for a stimulation of the dopamine system by binding to the allosteric site might then be at least 10 times lower, that is 2.5 to 15 mg per day. A general appropriate oral dosage according to the invention is from 1 to 20 mg per day, given once daily or divided in two equal doses. Suitable subcutaneous or intramuscular doses are only slightly lower, whereas intravenous doses are approximately 10 times less than the oral dosage.
Examples of disorders treatable according to the invention are:
Parkinson's disease in early stages, before introducing L-dopa or directly acting dopamine receptor agonists, or together with low doses of the same. Restless legs, again either monotherapy or combinations as above. Akathisia. Dystonias. Mental fatigue, associated with high age, stroke, postencephalitic and posttraumatic conditions. Attention-deficit disorders (ADHD). Autism spectrum disorders. Lapses of consciousness, e g narcolepsy and petit mal epilepsy, syncope. Sleeping disorders, e g hypersomnia, sleep apnea, attacks of sleep induced e g by dopamine receptor agonists. Emesis and nausea, induced by dopamine agonists, other drugs, motion sickness and other causes. Dopamine hypofuction induced by antipsychotic drugs, e g extrapyramidal symptoms, depression, loss of initiative, cognitive deficits (combined treatment).
However, the present invention has important additional clinical implications. In addition to the above-mentioned disorders, which are characterized by a hypofunction of dopamine, a large number of disorders rather seem to be due to a lack of stability of complex neural circuits controlled by dopamine neurons. In such circuits a dopamine stabilizer appears to be able to restore the stability even in cases where the primary cause of instability is located outside the dopamine system. A typical example is Huntington's disease, which is primarily due to degeneration of gabaergic neurons in the basal ganglia. Nevertheless, as shown in clinical trials using both (-)-OSU6162 and ACR16, these stabilizers can improve both signs of overactivity, e g chorea, and underfunctions, e g cognitive failure and depression. Such a stabilizing action is assumed to be due to blockade of mutually antagonistic subpopulations of the dopamine D2 receptor, such as the autoreceptors and the postsynaptic heteroreceptor. It has been assumed that autoreceptor is more readily blocked by a dopamine stabilizer than the postsynaptic receptor, and there is some evidence for that. However, the present invention introduces a new player, that is the allosteric site, on which the stabilizers can act in much lower concentrations than in the case of the orthosteric sites to counterbalance the hypofunction caused by blockade of the postsynaptic heteroreceptor. Obviously this should lead to the existence of a therapeutic window, where the optimum dosage should lie between those needed for a functionally relevant degree of binding to the allosteric and orthosteric sites, respectively. The consequence should then be that the optimum dosage for stabilization should lie between those needed for binding to the two sites, and thus lower than those previously assumed, which were based only on the data obtained from studies of the orthosteric sites. Thus it does not seem unlikely that the doses tried so far have been to high in relation to the therapeutic window, and thus that the benefit of the treatment has been underestimated. Thus the invention leads to the conclusion that future clinical trials in disorders characterized by instability of neural circuits must avoid too high doses by taking into account the probable existence of a therapeutic window. A proposed average dose intended for stabilization could be 25 - 50 mg per day, given once or preferably divided in two equal doses.
Examples of disorders, where an instability of neural circuits appear to be of crucial importance and where dopamine stabilizers in adequate dosage would be expected to be beneficial, are:
   Schizophrenia, with positive or negative/cognitive deficit symptoms, or both. Other psychoses and paranoid conditions. Manic-depressive disorder. Huntington's disease (both chorea and mental symptoms, e g cognitive deficits). Tics, Tourette's disease, hiccup. Dyskinesias induced by L-dopa and other dopamine-receptor agonists. Tardive dyskinesias, induced by long-term treatment with dopamine receptor antagonists. Drug abuse and addiction (central stimulants, central depressants, alcohol, cannabis, opiates, nicotine). Addiction to gambling, certain foodstuffs etc. Emotional disorders, aggressiveness, pathological impulsiveness. Emotional disturbances induced by severe pain(treatment combined with analgesics). Anxiety disorders, panic disorders, generalized anxiety disorder, obsessive-compulsive disorder. Combined treatment with antipsychotic drugs, serving to alleviate their adverse effects (extrapyramidal, executive, cognitive, emotional).
   The above subdivision into two categories of disorders which would be expected to benefit from the present invention, aims to guide the search for an optimal dosage. However, it should be kept in mind that there is no clear demarcation line between the two categories.
   One could consider a third category of disorders, which would be characterized by a pure hyperfunction of the dopamine system. However, the existence of such conditions may be questioned. Even if, for example, abuse of central stimulants initially leads to dopamine hyperfunction, addicts at the time of asking for medical help also suffer from instability and/or overt weakness of the dopamine system. As to spontaneous disorders, schizophrenia with predominantly positive symptoms, indicating dopamine hyperfunction will also almost invariably be accompanied by signs of dopamine hypofunction (negative symptoms and cognitive deficits). The same thing is true of mania, and so forth.
   The pharmaceutical composition according to the invention, used according to the invention or produced according to the invention may also comprise other substances, such as an inert vehicle, or pharmaceutical acceptable adjuvants, carriers, preservatives etc., which are well known to persons skilled in the art.

### Examples

The effect of (-)-OSU6162 on the incorporation of GTPgammaS³⁵ in the membranes of hD₂ₗ-transfected CHO cells was investigated. In the absence of dopamine the compound exerted a slight but significant stimulating action, suggesting a weak partial agonism. In the presence of dopamine, low concentrations (10 to 100 nM) enhanced the stimulating action of dopamine. This enhancing effect was reversed by higher concentrations of (-)-OSU6162 in a complex biphasic manner. The dopamine-enhancing action is proposed to be mediated by binding to an allosteric site with high affinity and the inhibitory component by a low-affinity binding to the orthosteric site of the dopamine receptor.

### Methods

Materials. (-)-OSU 6261 was obtained from Dr. Clas Sonesson (University of Gothenburg, Gothenburg, Sweden). Dopamine and GDP were purchased from Sigma Chemical Co. (St Louis MO). Unlabeled GTPgammaS was purchased from Roche (Indianapolis ,IN). All drugs were dissolved in deionized water and prepared daily. GTPgammaS³⁵ (1250 Ci/mmol) was purchased from NEN Life Science Products (Boston MA).
CHO-hD₂ₗ cells were grown to near confluence in T-150 flasks, and cells harvested, and membranes prepared as previously described (Lahti et al 1992). Membranes were stored at -80° C at a concentration of 1 mg protein/ml buffer.
Dopamine-stimulated GTPgammaS³⁵ incorporation was determined using the following procedure: in a buffer containing 20 mM HEPES, 5 mM Mg Cl2, 100 mM NaCl, at pH 7.5, membranes (60 µg/ml) were incubated with vehicle, drugs and 3 µM GDP for 30 min at 30° C, total volume was 1.1 ml. Then were added 30 microliter of GTPgammaS³⁵ (160 pM) and the incubation continued for another 30 min as before. The reaction was stopped by filtering the samples with a Brandel harvester on Whatmann GF/B filters. Filters were placed in liquid scintillation vials containing 10 ml of BCS scintillation fluid and counted the next day. All samples were run in triplicate.

### Results

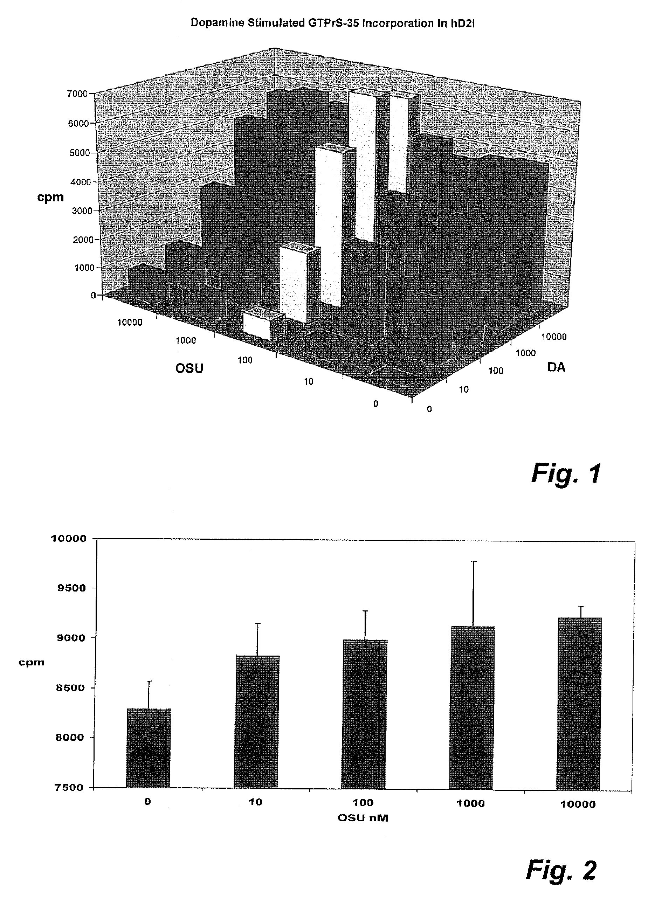

Figure 1 presents an overview of the effect of various concentrations of dopamine and (-)-OSU6162 on the incorporation of GTPgammaS³⁵. Following addition of dopamine alone a sigmoid-shaped curve is apparent, with a ceiling effect between 1,000 and 10,000 nM dopamine concentrations. A similar result was obtained in another experiment under the same experimental conditions.
In the absence of dopamine, 100 to 10,000 nM concentrations of (-)-OSU6162 increased the incorporation of GTPgammaS³⁵ by 8 to 11 percent (Figure 2, data extracted from Figure 1).
When dopamine and (-)-OSU6162 concentrations were varied simultaneously, the two agents showed a complex interaction on GTPgammaS³⁵ incorporation (Figure 3, data extracted from Figure 1). At low concentrations of dopamine (10 to 100 nM), low concentrations of (-)-OSU6162 (10 to 100 nM) caused an increased incorporation of GTPgammaS³⁵. However, this increase was no longer seen after higher concentrations of (-)-OSU6162 (1,000 to 10,000 nM). At higher dopamine concentrations (1,000 and 10,000 nM) variations in (-)-OSU6162 levels yielded similar, though flatter activity curves.

### Discussion

The data above demonstrate both stimulating and inhibitory effects of (-)-OSU6162 on the activity of dopamine D₂ receptors, expressed as GTPgammaS³⁵ incorporation. When given alone, (-)-OSU6162 was found to exert a weak stimulating effect on GTPgammaS³⁵ incorporation, suggesting that this agent is a partial agonist on dopamine D₂ receptors. In support of this hypothesis this compound is chemically closely related to 3PPP, whose enantiomers possess agonist properties on dopamine D₂/D₃ receptors. In addition (-)-OSU6162 has a behavioural profile fitting in with a weak partial agonism on dopamine D₂ receptors. Seeman and Guan (2006) also showed the mild agonist effect of (-)-OSU6162 on GTPgammaS³⁵ incorporation into cloned CHO cells with the D₂long dopamine receptor, but failed to demonstrate the complex interaction of varied concentrations of (-)- OSU6162 on the agonist effects. So far, however, direct in-vivo testing of (-)-OSU6162 has failed to demonstrate any dopamine receptor agonism (Natesan et al. 2006). The most likely explanation of the available data appears to be that (-)-OSU6162 is an albeit weak partial agonist on dopamine D₂ receptors. Probably such weak agonism is more easily detectable under in-vitro conditions, because a complete absence of dopamine can hardly be reached under in-vivo conditions.
When various concentrations of dopamine and (-)-OSU6162 were studied in combination, (-)-OSU8162 was found to have a biphasic effect on dopamine D₂ receptor activity. A stimulating action, enhancing the effect of dopamine, showed up after low concentrations of (-)-OSU6162, but this enhancing effect was reversed by higher concentrations, which thus seemed to antagonize the effect of dopamine.
The following interpretation would probably best fit in with previous knowledge about (-)-OSU6162. Both in-vitro binding and in-vivo data support the view that (-)-OSU6162 is an antagonist on dopamine D₂ receptors, albeit with a weak intrinsic activity, so far detectable only under in-vitro conditions. The antidopaminergic component induced by high concentrations of (-)-OSU6162 under the present conditions may be explained accordingly. This fits in with the fairly high Ki of the compound (in the micromolar region) in the previous in-vitro binding experiments (Sonesson et al. 1994).
The stimulation induced by low (-)-OSU6162 concentrations could then be due to binding to an allosteric site, leading to an enhanced action of dopamine, and possibly to the weak stimulation of the receptor observed even in the absence of dopamine. The binding of this compound to an allosteric site might lead to a conformational change of the receptor molecule, and this in turn could have a number of different consequences: a) an increased affinity to dopamine, b) an increased responsiveness to dopamine by some other mechanism, c) an increased constitutive activity of the receptor, or d) any combination between these possibilities. Alternative explanations may need to be considered, involving for example, binding to some adjacent protein which in one way or another might influence the dopamine receptor.
The complex mechanisms proposed above could perhaps be looked upon as a model for receptor stabilization: simultaneous occupancies by one and the same agent on allosteric and orthosteric sites, counterbalancing each other without necessarily disturbing the baseline receptor activity, should lead to a stabilizing action, because the amplitude of responses to endogenous dopamine release would be dampened, due to a reduced availability of binding sites. However, the extrapolation of data obtained in an artificial in-vitro system to in-vivo conditions must be regarded as tentative only.
The present experiments were performed on the long splice variant of the human D₂ receptor. It remains to be seen how the short variant will behave in this experimental model. There is a functional difference between the two variants at least in so far as auto- versus hetero-receptors are concerned: the former seem to belong predominantly to the short variant, whereas the latter are mixed (Khan et al 1998, Centonze et al 2002). The heteroreceptors are either synaptic or extrasynaptic. An attractive hypothesis would be that the short variant is predominantly extrasynaptic, regardless of whether it is an auto- or heteroreceptor, whereas the long variant would be primarily located to the synaptic cleft. The two variants would then be geared to different neurotransmitter concentrations. There is good evidence that the autoreceptors are more responsive to low transmitter concentrations than the heteroreceptor (Carlsson and Carlsson 2006). Moreover, postsynaptic dopamine receptors rendered extrasynaptic following degeneration of dopamine neurons become supersensitive. It would be interesting to compare the two splice variants in the present experimental model. In general, in-vitro binding experiments have so far shown them to behave equally, using a large number of antidopaminergic agents (Leysen et al 1993). However, one study has reported somewhat higher affinities of atypical antipsychotics to the short variant (Malmberg et al 1993).
The possible existence of an endogenous ligand for the proposed allosteric site could be a matter for speculation. Such a ligand could be released along with dopamine as a co-transmitter or from an adjacent cell. It could either enhance or dampen the function of dopamine and thus serve as an amplifier or stabilizer. The simultaneous existence of both amplifying and stabilizing allosteric ligands may also be considered.

### References

Carlsson A, Carlsson ML (2006) A dopaminergic deficit hypothesis of schizophrenia: the path to discovery. Dialogues Clin Neurosci 8(1):137-42
Carlsson ML, Carlsson A, Nilsson M.(2004) Schizophrenia: from dopamine to glutamate and back. Curr Med Chem. Feb;11(3):267-77 Centonze D, Usiello A, Gubellini P, Pisani A, Borrelli E, Bernardi G, Calabresi P (2002) Dopamine D2 receptor-mediated inhibition of dopaminergic neurons in mice lacking D2l receptors. Neuropsychopharmacology Nov;27(5):723-6 Gefvert O, Lindström L, Dahlbäck O, Sonesson C, Waters N (2000) (-)-OSU6162 induces a rapid onset of antipsychotic effect after a single dose. A double-blind placebo-controlled study. Nord J Psychiat pp. 93-94. Abstract Khan ZU, Mrzljak L, Gutierrez A, de la Calle A, Goldman-Rakic PS (1998) Prominence of the dopamine D2 short isoform in dopaminergic pathways. Proc Natl Acad Sci U S A. Jun 23;95(13):7731-6 Lahti RA, Figur LM, Piercey MF, Ruppel PL, Evans DL (1992) Intrinsic activity determinations at the dopamine D2 guanine nucleotide-binding protein-coupled receptor: utilization of receptor state binding affinities. Mol Pharmacol Sep;42(3):432-8 Leysen JE, Gommeren W, Mertens J, Luyten WH, Pauwels PJ, Ewert M, Seeburg P (1993) Comparison of in vitro binding properties of a series of dopamine antagonists and agonists for cloned human dopamine D2S and D2L receptors and for D2 receptors in rat striatal and mesolimbic tissues, using [125I] 2'-iodospiperone. Psychopharmacology (Berl) 110(1-2):27-38 Malmberg A, Jackson DM, Eriksson A, Mohell N (1993) Unique binding characteristics of antipsychotic agents interacting with human dopamine D2A, D2B, and D3 receptors. Mol Pharmacol May;43(5):749-54
Natesan S, Svensson KA, Reckless GE, Nobrega JN, Barlow KB, Johansson AM, Kapur S (2006) The dopamine stabilizers (-)-OSU6162 and ACR16 show high in vivo D2 receptor occupancy, antipsychotic- like efficacy and low potential for motor side effects in the rat. J Pharmacol Exp Ther. Aug;318(2):810-8
Neu H, Hartvig P, Torstenson R, Fasth KJ, Sonesson C, Waters N, Carlsson A, Tedroff J, Langstrom B (1997) Synthesis of [11C-methyl]-(-)-OSU6162, its regional brain distribution and some pharmacological effects of (-)-OSU6162 on the dopaminergic system studied in the rhesus monkey by positron emission tomography. Nucl Med Biol Aug;24(6):507-11
Sonesson C, Lin CH, Hansson L, Waters N, Svensson K, Carlsson A, Smith MW, Wikstrom H (1994) Substituted (S)-phenylpiperidines and rigid congeners as preferential dopamine autoreceptor antagonists: synthesis and structure-activity relationships. J Med Chem Aug 19;37(17):2735-53

## Claims

1. Use of a dopamine stabilizing substance selected from the group consisting of:
compounds of formula I 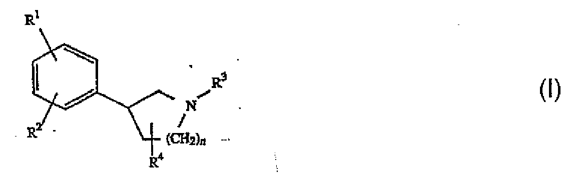
wherein:
R¹ and R² are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
R³ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
R⁴ and R are independently selected from hydrogen, CF₃.CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or -(CH₂)ₘ-R⁵ where m is 1-8;
R⁵ is phenyl, phenyl substituted with CN, CF₃, CH₂CF₃, C₁ -C₈ alkyl, C₃-C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷ ; and
R⁶ and R⁷ are independently H, C₁-C₈ alkyl, C₃-C₈ cycloalkyl, C₄-C₉ cycloalkyl-methyl, C₂-C₈ alkenyl or C₂-C₈ alkynyl;
with the provisos that (i) when R¹ is CN, R² and R⁴ are H, and R³ is n-Pr then the compound is a pure enantiomer: and (ii) when R¹ or R² is OH, or CONH₂ then R⁴ is not H or methyl; compounds of formula II 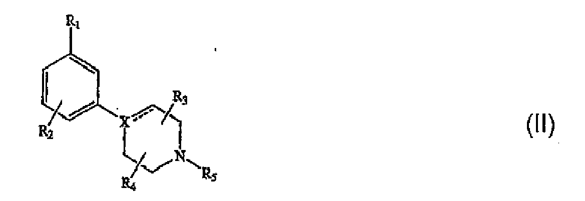 wherein:
R₁ is selected from the group consisting of OSO₂CF₃, OSO₂CH₃, SOR₃, SO₂R₃, COCH₃, and COCH₂CH₃, wherein R₃ is as defined below;
R₂ is selected from the group consisting of C₂-C₄ branched or unbranched alkyls, terminal allyl, CH₂CH₂OCH₃, CH₂CH₂CH₂F, CH₂CF₃, 3,3,3-trifluoropropyl, and 4,4,4-trifluorobutyl,
R₃ is selected from the group consisting of C1-C₃ alkyls, CF₃, and N(CH₃)₂;.
wherein the compound of formula II does not have a high binding affinity to sigma receptors
and pharmaceutically acceptable salts thereof for the manufacture of a medicament or a pharmaceutical composition for treatment of a neurological or psychiatric disorder **characterized by** a hypofunction of the dopamine system, wherein said substance is to be administered in an oral, subcutaneous or intramuscular daily dose of 1-20 mg or in an intravenous daily dose of 0.1-2 mg.

2. Use according to claim 1, wherein said neurological or psychiatric disorder **characterized by** a hypofunction of the dopamine system is selected from the group consisting of Parkinson's disease in early stages; restless legs; akathisia; dystonias; mental fatigue associated with high age, stroke, postencephalitic or posttraumatic conditions; attention-deficit disorders (ADHD); autism spectrum disorders; lapses of consciousness including narcolepsy, petit mal epilepsy and syncope; sleeping disorders including hypersomnia, sleep apnea, and attacks of sleep induced by dopamine receptor agonists; emesis and nausea; dopamine hypofuction induced by antipsychotic drugs.

3. Use according to claim 1 or 2, wherein the oral, subcutaneous or intramuscular daily dose is 2.5 to 15 mg.

4. Use of a dopamine stabilizing substance selected from the group consisting of:
compounds of formula I 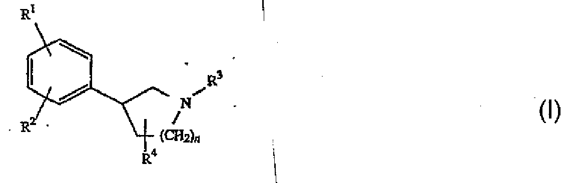
wherein:
R¹ and R² are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
R³ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
R⁴ and R are independently selected from hydrogen, CF₃.CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or -(CH₂)ₘ-R⁵ where m is 1-8;
R⁵ is phenyl, phenyl substituted with CN, CF₃, CH₂CF₃, C₁ -C₈ alkyl, C₃ - C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷ ; and
R⁶ and R⁷ are independently H, C₁-C₈ alkyl, C₃-C₈ cycloalkyl, C₄-C₉ cycloalkyl-methyl, C₂-C₈ alkenyl or C₂-C₈ alkynyl; with the provisos that (i) when R¹ is CN, R² and R⁴ are H, and R³ is n-Pr then the compound is a pure enantiomer: and (ii) when R¹ or R² is OH, or CONH₂ then R⁴ is not H or methyl;
compounds of formula II 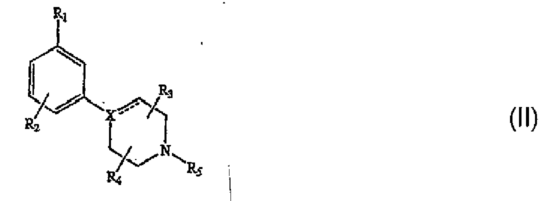 wherein:
R₁ is selected from the group consisting of OSO₂CF₃, OSO₂CH₃, SOR₃, SO₂R₃, COCH₃, and COCH₂CH₃, wherein R₃ is as defined below;
R₂ is selected from the group consisting of C₂-C₄ branched or unbranched alkyls, terminal allyl, CH₂CH₂OCH₃, CH₂CH₂CH₂F, CH₂CF₃, 3,3,3-trifluoropropyl, and 4,4,4-trifluorobutyl,
R₃ is selected from the group consisting of C1-C₃ alkyls, CF₃, and N(CH₃)₂;
wherein the compound of formula II does not have a high binding affinity to sigma receptors
and pharmaceutically acceptable salts thereof for the manufacture of a medicament or a pharmaceutical composition for treatment of a disorder caused by instability of neural circuits, wherein said substance is to be administered in an oral, subcutaneous or intramuscular daily dose of 25-50 mg.

5. Use according to claim 4, wherein said disorder caused by instability of neural circuits is selected from the group consisting of schizophrenia; other psychoses and paranoid conditions; manic-depressive disorder; Huntington's disease; Tics; Tourette's disease; hiccup; dyskinesias induced by L-dopa and other dopamine-receptor agonists; tardive dyskinesias induced by long-term treatment with dopamine receptor antagonists; drug abuse and addiction; addiction to gambling; addiction to certain foodstuffs etc; emotional disorders including aggressiveness and pathological impulsiveness; emotional disturbances induced by severe pain; anxiety disorders including panic disorders, generalized anxiety disorder and obsessive-compulsive disorder; and adverse effects, including extrapyramidal, executive, cognitive and emotional caused by antipsychotic drugs.

6. Use according to any one of the preceding claims, wherein a compound of formula I or a pharmaceutically acceptable salt thereof is used in the form of a pure enantiomer.

7. Use according to any one of the preceding claims, wherein a compound of formula I or a pharmaceutically acceptalbe salt thereof is used, wherein R¹ is CN, OSO₂CF₃, or SO₂CH₃.

8. Use according to any one of the preceding claims, wherein R² is H and R³ is C₁₋₈ alkyl.

9. Use according to any one of the preceding claims, wherein R² is H and R³ is n-propyl.

10. Use according to any one of the preceding claims, wherein R⁴ is H.

11. Use according to any one of the preceding claims, wherein a compound of formula I or a pharmaceutically acceptalbe salt thereof is used, wherein R¹ is 3-OH, R² is H, R³ is n-propyl and R⁴ is C₂₋₈ alkyl.

12. Use according to any one of the preceding claims, wherein said substance is S-(-)-3-[3-methylsulfonylphenyl]-1-propylpiperidine).

13. Use according to any one of claims 1-5, wherein a compound of formula II or a pharmaceutically acceptalbe salt thereof is used, wherein R₁ is selected from the group consisting of OSO₂CF₃, OSO₂CH₃, SO₂CH₃, SO₂CF₃, COCH₃, and SO₂N(CH₃)₂.

14. Use according to claim 13, wherein R₁ is selected from the group consisting of SO₂CF₃, SO₂CH₃, and COCH₃.

15. Use according to any one of claims 1-5, 13 or 14, wherein a compound of formula II or a pharmaceutically acceptalbe salt thereof is used, wherein R₂ is selected from the group consisting of n-propyl and ethyl.

16. Use according to any one of claims 1-5 or 13-15, wherein a compound of formula II or a pharmaceutically acceptalbe salt thereof is used and said compound is 4-(3-methanesulfonylphenyl)-1-propyl-piperidine.

17. Use according to any one of the preceding claims, wherein the daily does is given as one dose a day.

18. Use according to any one of claims 1-16, wherein the daily does is divided into two equal doses.

19. A method for treating a neurological or psychiatric disorder **characterized by** a hypofunction of the dopamine system comprising administering to a patient in need thereof a dopamine stabilizing substance selected from the group consisting of:
compounds of formula I 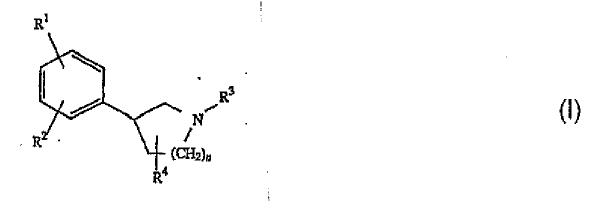
wherein:
R¹ and R² are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
R³ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
R⁴ and R are independently selected from hydrogen, CF₃.CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or -(CH₂)ₘ-R⁵ where m is 1-8;
R⁵ is phenyl, phenyl substituted with CN, CF₃, CH₂CF₃, C₁ -C₈ alkyl, C₃-C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷ ; and
R⁶ and R⁷ are independently H, C₁-C₈ alkyl, C₃-C₈ cycloalkyl, C₄-C₉ cycloalkyl-methyl, C₂-C₈ alkenyl or C₂-C₈ alkynyl;
with the provisos that (i) when R¹ is CN, R² and R⁴ are H, and R³ is n-Pr then the compound is a pure enantiomer: and (ii) when R¹ or R² is OH, or CONH₂ then R⁴ is not H or methyl; compounds of formula II 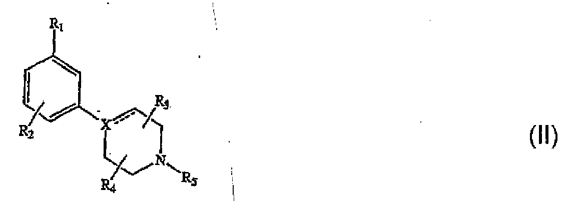 wherein:
R₁ is selected from the group consisting of OSO₂CF₃, OSO₂CH₃, SOR₃, SO₂R₃, COCH₃, and COCH₂CH₃, wherein R₃ is as defined below;
R₂ is selected from the group consisting of C₂-C₄ branched or unbranched alkyls, terminal allyl, CH₂CH₂OCH₃, CH₂CH₂CH₂F, CH₂CF₃, 3,3,3-trifluoropropyl, and 4,4,4-trifluorobutyl,
R₃ is selected from the group consisting of C1-C₃ alkyls, CF₃, and N(CH₃)₂;.
wherein the compound of formula II does not have a high binding affinity to sigma receptors and pharmaceutically acceptable salts thereof,
in an oral, subcutaneous or intramuscular daily dose of 1-20 mg or in an intravenous daily dose of 0.1-2 mg.

20. The method of claim 19, wherein said neurological or psychiatric disorder **characterized by** a hypofunction of the dopamine system is selected from the group consisting of Parkinson's disease in early stages; restless legs; akathisia; dystonias; mental fatigue associated with high age, stroke, postencephalitic or posttraumatic conditions; attention-deficit disorders (ADHD); autism spectrum disorders; lapses of consciousness including narcolepsy, petit mal epilepsy and syncope; sleeping disorders including hypersomnia, sleep apnea, and attacks of sleep induced by dopamine receptor agonists; emesis and nausea; dopamine hypofuction induced by antipsychotic drugs.

21. The method of claim 19, wherein the oral, subcutaneous or intramuscular daily dose is 2.5 to 15 mg.

22. A method for treating a disorder caused by instability of neural circuits comprising administering to a patient in need thereof a dopamine stabilizing substance selected from the group consisting of: compounds of formula I 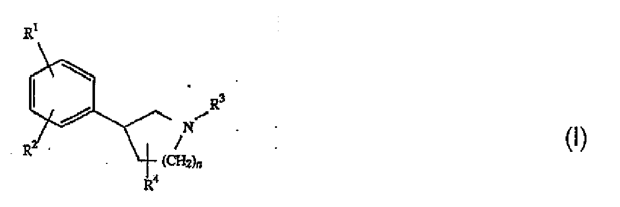 wherein:
R¹ and R² are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
R³ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
R⁴ and R are independently selected from hydrogen, CF₃.CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorabutyl or -(CH₂)ₘ-R⁵ where m is 1-8;
R⁵ is phenyl, phenyl substituted with CN, CF₃, CH₂CF₃, C₁ -C₈ alkyl, C₃ - C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷ ; and
R⁶ and R⁷ are independently H, C₁-C₈ alkyl, C₃-C₈ cycloalkyl, C₄-C₉ cycloalkyl-methyl, C₂-C₈ alkenyl or C₂-C₈ alkynyl;
with the provisos that (i) when R¹ is CN, R² and R⁴ are H, and R³ is n-Pr then the compound is a pure enantiomer: and (ii) when R¹ or R² is OH, or CONH₂ then R⁴ is not H or methyl;
compounds of formula II 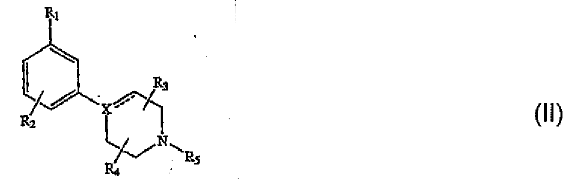 wherein:
R₁ is selected from the group consisting of OSO₂CF₃, OSO₂CH₃, SOR₃, SO₂R₃, COCH₃, and COCH₂CH₃, wherein R₃ is as defined below;
R₂ is selected from the group consisting of C₂-C₄ branched or unbranched alkyls, terminal allyl, CH₂CH₂OCH₃, CH₂CH₂CH₂F, CH₂CF₃, 3,3,3-trifluoropropyl, and 4,4,4-trifluorobutyl,
R₃ is selected from the group consisting of C₁-C₃ alkyls, CF₃, and N(CH₃)₂;.
wherein the compound of formula II does not have a high binding affinity to sigma receptors and pharmaceutically acceptable salts thereof,
in an oral, subcutaneous or intramuscular daily dose of 25-50 mg.

23. The method of claim 22, wherein said disorder caused by instability of neural circuits is selected from the group consisting of schizophrenia; other psychoses and paranoid conditions; manic-depressive disorder; Huntington's disease; Tics; Tourette's disease; hiccup; dyskinesias induced by L-dopa and other dopamine-receptor agonists; tardive dyskinesias induced by long-term treatment with dopamine receptor antagonists; drug abuse and addiction; addiction to gambling; addiction to certain foodstuffs etc; emotional disorders including aggressiveness and pathological impulsiveness; emotional disturbances induced by severe pain; anxiety disorders including panic disorders, generalized anxiety disorder and obsessive-compulsive disorder; and adverse effects, including extrapyramidal, executive, cognitive and emotional caused by antipsychotic drugs.

24. The method of claim 19 or 22, wherein a compound of formula I or a pharmaceutically acceptable salt thereof is used in the form of a pure enantiomer.

25. The method of claim 19 or 22, wherein a compound of formula I or a pharmaceutically acceptalbe salt thereof is used, wherein R¹ is CN, OSO₂CF₃, or SO₂CH₃.

26. The method of claim 25, wherein R² is H and R³ is C₁₋₈ alkyl.

27. The method of claim 26, wherein R² is H and R³ is n-propyl.

28. The method of claim 275, wherein R⁴ is H.

29. The method of claim 19 or 22, wherein a compound of formula I or a pharmaceutically acceptalbe salt thereof is used, wherein R¹ is 3-OH, R² is H, R³ is n-propyl and R⁴ is C₂₋₈ alkyl.

30. The method of claim 19 or 22, wherein said substance is S-(-)-3-[3-methylsulfonylphenyl]-1-propylpiperidine).

31. The method of claim 19 or 22, wherein a compound of formula II or a pharmaceutically acceptalbe salt thereof is used, wherein R₁ is selected from the group consisting of OSO₂CF₃, OSO₂CH₃, SO₂CH₃, SO₂CF₃, COCH₃, and SO₂N(CH₃)₂.

32. The method of claim 31, wherein R₁ is selected from the group consisting of SO₂CF₃, SO₂CH₃, and COCH₃.

33. The method of claim 19 or 22, wherein a compound of formula II or a pharmaceutically acceptalbe salt thereof is used, wherein R₂ is selected from the group consisting of n-propyl and ethyl.

34. The method of claim 19 or 22, wherein a compound of formula II or a pharmaceutically acceptalbe salt thereof is used and said compound is 4-(3-methanesulfonylphenyl)-1-propyl-piperidine.

35. The method of claim 19 or 22, wherein the daily does is given as one dose a day.

36. The method of claim 19 or 22, wherein the daily does is divided into two equal doses.
